Europäisches Patentamt

European Patent Office

Office européen des brevets

(11) Publication number: **0 316 146**
**A1**

# EUROPEAN PATENT APPLICATION

(21) Application number: 88310516.5

(22) Date of filing: 09.11.88

(51) Int. Cl.4: **A61K 47/00 , A61K 31/715**

(30) Priority: 10.11.87 US 119188
07.04.88 US 178472

(43) Date of publication of application:
**17.05.89 Bulletin 89/20**

(84) Designated Contracting States:
**DE FR GB SE**

(71) Applicant: **NATIONAL BIOSOURCE, INC.**
**520 E. Montecito Street Suite G**
**Santa Barbara California 93108(US)**

(72) Inventor: **Kuhrts, Eric H.**
**903 Park Lane**
**Santa Barbara California 93108(US)**

(74) Representative: **Perry, Robert Edward et al**
**GILL JENNINGS & EVERY 53-64 Chancery**
**Lane**
**London WC2A 1HN(GB)**

(54) A powder drink mix for reducing serum cholesterol.

(57) A powder which can be mixed in water and orally ingested, the resulting solution being effective in reducing serum cholesterol levels, is disclosed. The powder mixture contains powdered guar gum and a food-grade acid such as citric acid. The powder is mixed in water immediately prior to being ingested. The food-grade acid extends the time in which the fiber congeals, allowing the powder to be mixed with water and ingested before gelation occurs as well as providing enhanced dispersibility of the powdered guar gum in water. The guar gum powdered drink mix lowers serum cholesterol levels when taken at regular intervals over a period of time.

EP 0 316 146 A1

## A POWDER DRINK MIX FOR REDUCING SERUM CHOLESTEROL

### Background of the Invention

Gel-forming fibers, such as guar gum, have been disclosed as useful in reducing serum cholesterol in articles such as the one by Superko, R.H., Decreasing Blood Cholesterol Levels with a Dietary Additive: An Additional Approach to Diet And Drugs, Cardiovascular Reviews and Reports, 6:No. 11, p. 1253 (Nov. 1985). This study of the effects of guar gum on serum cholesterol levels showed reductions in total cholesterol ranging from the statistically insignificant 3.4% to 16.6%.

The use of gel-forming fibers in combination with mineral carbonates for reducing serum cholesterol levels in orally-administrable capsules which are orally ingested is disclosed in the application entitled "METHOD AND COMPOSITION FOR REDUCING SERUM CHOLESTEROL", Serial No. 871,715 to Day et al., the teaching of which is incorporated herein by reference. That application, and the references cited therein, indicate the relationship between high serum cholesterol levels and heart disease. Due to the difficulty in getting people to change their dietary and exercise habits to lower serum cholesterol, a need has arisen for other palatable dosage forms of these non-prescription cholesterol reducing agents, such as, for example, a conveniently-administered powder mix.

Heretofore, it has been difficult to make a guar gum drink-mix, because of the tendency of the guar gum to gel rapidly when placed in water. Also problematic was the difficulty in dispersing guar gum in water. The guar gum powder, when placed in water, has a tendency to clump together rather than disperse uniformly throughout the mixture. Therefore, guar gum has previously only been available in solid dosage forms such as Bio GuarTM capsules manufactured by National BioSource.

### Summary of the Invention

The present invention provides a palatable powdered drink mix which when orally administered, is effective in reducing serum cholesterol levels. It is an object of the invention to provide a powder containing a gel-forming fiber which can be mixed with water yet remain in solution for a period of time sufficient to enable the person to drink the mixture, prior to gelation of the gel-forming fiber. It is a further object of this invention to provide a guar gum powder which is dispersable in water without clumping. It is another object of this invention to provide a palatable liquid for reducing serum cholesterol levels in humans which provides superior results. The composition shows a significant reduction in a subject's total cholesterol level if taken at a dosage of 5 grams three times per day over a six-week period.

### Detailed Description of the Invention

The primary consideration with any substance of therapeutic value is that the substance be taken in the necessary dosage with the necessary frequency to accomplish its therapeutic purpose. Gel-forming fibers, in order to be effective in the reduction of serum cholesterol, must be administered several times daily over a period of time in order for a significant reduction in serum cholesterol to be achieved. Just as a prescription of diet and exercise is ineffective for the reduction of cholesterol if the patient does not alter his eating or exercise habits, a protocol of over-the-counter dietary supplements will be ineffective if they are unpleasant or difficult to take.

Since regular and repeated administration of the gel-forming fiber is required for the desired therapeutic effect, an unpleasant or inconvenient method of administration will probably not achieve the desired results, as patient compliance is a necessary factor in achieving these results. A palatable powder drink mix which contains a gel-forming fiber effective in the reduction of serum cholesterol is desirable because patient non-compliance will be minimized.

A powdered drink mix containing gel-forming fiber can incorporate as much fiber in one teaspoon as is found in 10 capsules containing the gel-forming fiber. The problem, as discussed above, is getting the powdered gel-forming fiber to dissolve in water without clumping or gelling, thereby making the drink mix palatable as well as easy and pleasant to take.

The addition of a food-grade acid (an acid that is suitable for consumption by humans such as, for example, citric acid) in powder form to a powdered gel-forming fiber such as, for example, guar gum, enhances the product's palatability. The food-grade acid delays the gelation of the guar gum, enabling the resulting mixture to be ingested conveniently. Also, the powder disperses in water, rather than agglomerating and forming powder balls which would obviously make the mixture difficult to drink.

In addition to the powder mix being easier to administer, the powder mix is more convenient to use. The powder can be packaged in single dosage (5 grams) foil packets or canisters containing a multiple-dosage quantity of the powder mix.

The powdered drink mix has another advantage in that it can be flavored to make it more pleasant to the taste. Furthermore, some patients may find swallowing capsules or tablets difficult. The present invention provides a convenient alternative dosage form which will improve patient compliance. With all the advantages discussed herein, the powdered drink mix offers a significant advance over other formulations using gel-forming fibers for the reduction of serum cholesterol levels, a significant and potentially serious health problem in this country as discussed below.

Coronary heart disease is still the number one cause of death in the United States. Elevated serum cholesterol is one of the major causes of coronary artery disease. In fact, severe coronary heart disease can result from high blood cholesterol levels in the absence of any other contributory risk factors.

The lipids in plasma of major clinical importance are cholesterol and triglycerides. Cholesterol is always present as a major ingredient in atherosclerotic plaque, along with fatty acids, esters of cholesterol, phosphatids, neutral fats and dihydrocholesterol.

Cholesterol is not miscible with water. To carry it in the blood, it is combined or repackaged with protein. The combination of cholesterol and protein is called a lipoprotein. Very low-density lipoproteins (VLDL or pre-beta-lipoproteins), carry endogenously synthesized triglycerides, which are removed by muscle, heart, adipose tissue, and other sites. Major remnants of VLDL metabolism are low-density lipoproteins (LDL or beta-lipoproteins). LDLs are catabolized by a mechanism involving receptors in cell membranes, but the major organ binding sites besides the liver are not well defined. It is the LDLs which contain the greatest percentage of cholesterol. These particles, when present in excess in the bloodstream, are deposited in the tissues and form a major part of the build-up in the arterial wall to form atherosclerotic plaque which narrows the channels of the coronary arteries which furnish the major blood supply to the heart muscle. High density lipoproteins (HDL or alpha-lipoproteins) contain phospholipids and cholesterol complexed with apolipoproteins, the bulk of which differ from those found in VLDLs and LDLs. It is the HDLs which contain the greatest amount of protein and the smallest amount of cholesterol and are believed to take cholesterol away from cells and transport it back to the liver for processing or removal. In the post-absorptive state, a total plasma cholesterol concentration of 200 mg/dl is distributed very roughly as follows : VLDL, 10; LDL, 120; and, HDL, 50. Most of the plasma triglycerides above about 50 mg/dl will be found in VLDLs.

There is no absolute definition of hyperlipidemia. For biologic variables, upper limits such as the upper five or ten percent of the distribution within the population are often used but, for plasma cholesterol, these statistical limits are too high to be used clinically. Correlations between the cholesterol concentrations in young men in North America and incidence of premature ischemic heart disease indicate that an increasing risk can be detected when the cholesterol is higher than 220 mg/dl, a value close to the mean for men from 40 to 49 years of age in this population. Extrapolation of similar data from other populations suggest that cholesterol level at birth averages 60 mg/dl. Within one month, the average has risen to about 120 mg/dl and by the first year to 175 mg/dl. A second rise begins in the third decade and continues to about age fifty in men and somewhat later in women. In other populations this cholesterol rise in adulthood is far less prominent. It is therefore not to be considered necessarily physiologic. The age-related increases in cholesterol are associated mainly with the rise in LDL concentrations, the increases in triglycerides with a rise in VLDL. HDL concentrations in women average about 20% higher than in men. Estrogen tends to raise and androgens tend to lower HDL levels. The average serum cholesterol level for middle-aged adults in the U.S. is 215 mg/dl. The U.S. Health and Nutrition Examination Survey (1971-1974) indicates that the prevalence rate of a serum cholesterol level in excess of 260 mg/dl in the American male work force between the ages of 45 and 55 years is approximately 30%. (U.S. Department of Health and Human Services: Cardiovascular Primer for the Workplace, NIH Publication No. 81-2210, 75 (January, 1981)).

Evidence that decreasing LDL content in men with hypercholesterolemia results in a lower incidence of cardiovascular events has been greatly strengthened by the results of the Coronary Primary Prevention Trial-Lipid Research Clinic (LRC). This seven-year, randomized double blind trial conducted with 3,806 men convincingly demonstrated a significant reduction of myocardial infarction (MI) and sudden cardiac death in the group that achieved LDL reduction by means of a moderate diet in combination with cholestyramine. a bile acid-sequestering resin. This investigation further demonstrated that a 25% reduction in total plasma cholesterol (TC) concentration results in a 49% reduction in the frequency of myocardial infarction and sudden cardiac death. (Lipid Research Clinics Program: The Lipid Research Clinics Coronary Primary Prevention Trial Results: I. Reduction in Incidence of Coronary Heart Disease, and II. The Relationship of Reduction in Incidence of Coronary Heart Disease to Cholesterol Lowering, JAMA, 251: 351-374 (1984)). Accordingly, every 1% reduction in total cholesterol (TC) levels results in an approximately 2% reduction in risk of myocardial infarction.

In a broad aspect, the present invention is based upon the discovery that a powdered gel-forming fiber, such as for example, guar gum, when combined with a powdered food grade organic acid such as, for example, citric acid, tartaric acid, phosphoric acid, malic acid, or ascorbic acid, results in a palatable mixture when water is added. It is postulated that the food grade acid inhibits gelation of the fiber rendering the mixture easier to swallow after water is added. This solution, when taken at a dosage of 5 grams in 225-285 g water three times daily, has been shown to significantly reduce the serum cholesterol when taken over a period of time.

Other ingredients optionally added to the powdered mix can be for example ascorbic acid in an amount of from 4%-8% total weight of powder, a non-toxic mineral salt such as, for example, calcium carbonate, a flavoring such as, for example, natural orange flavor, potassium citrate, a sweetener such as, for example, aspartame (Nutra Sweet brand, Nutra Sweet is a registered trademark of G.D. Searle & Co.), and a coloring agent such as, for example, beta carotene. This mixture when dissolved in water is a pleasant-tasting orange-flavored drink which is easy to ingest.

The specific formulation used for testing was 81.4% by weight guar gum (manufactured by Agualon Company; Supercol G-3); 5.3% citric acid; 4.6% ascorbic acid; 2.88% natural orange flavor (Felton International FXA-3895); 2.54% calcium carbonate; 1.86% potassium citrate; 1% aspartame (Nutra Sweet brand, Nutra Sweet is a trademark of G.D. Searle & Co.); and .34% beta carotene. These percentages can be varied substantially depending upon the ingredients used. For example, if a fructose sweetener is used, the formulation may be approximately 50% sweetener. The fructose formulation requires more powder per dosage in order to ensure that an adequate amount of guar gum is in each dosage.

The specific formulation described in detail in the preceding paragraph, when administered in a dosage of 5 grams dissolved in 225-285 g water, three times daily for a period of six weeks, produced the results in Table 1 below.

**TABLE 1**

| SUBJECT | TCB | TCA | LDLB | LDLA | % CHANGE TC | % CHANGE LDL | RATIO CHANGE LDL/HDL |
|---------|-----|-----|------|------|-------------|--------------|----------------------|
| 1 | 411 | 319 | 325 | 243 | -22 | -25 | 5.5-4.6 |
| 2 | 279 | 149 | 155 | 85 | -46 | -45 | 3.5-3.3 |
| 3 | 242 | 208 | 162 | 136 | -14 | -16 | 5.8-4.9 |
| 4 | 226 | 136 | 156 | 105 | -39 | -33 | 5.0-4.3 |
| 5 | 380 | 329 | 300 | - | -13 | - | - |
| 6 | 258 | 225 | 183 | 158 | -13 | -13 | 7.9-6.2 |
| 7 | 262 | 214 | 180 | 151 | -18 | -16 | 5.8-5.1 |
| 8 | 228 | 202 | 174 | 157 | -11 | -10 | 4.6-4.1 |
| 9 | 265 | 231 | 198 | 166 | -13 | -16 | 5.0-4.5 |
| 10 | 308 | 264 | 225 | 196 | -14 | -13 | 4.6-4.2 |
| 11 | 277 | 237 | 210 | 197 | -14 | -6 | 7.2-7.6 |
| 12 | 358 | 331 | 291 | 264 | -8 | -11 | 10.8-8.8 |
| 13 | 316 | 231 | 238 | 157 | -27 | -34 | 4.25-2.7 |

Average Total Cholesterol Reduction:  19.38%

Average LDL Cholesterol Reduction:  19.83%

TCB = TOTAL CHOLESTEROL BEFORE

TCA = TOTAL CHOLESTEROL AFTER

Participant No. 5 dropped out of the study after two weeks. The percent change cholesterol for Participant No. 5 is the percent change over that two week period.

LDLB = LDL CHOLESTEROL BEFORE

LDLA = LDL CHOLESTEROL AFTER

RATIO CHANGE = LDL/HDL RATIO CHANGE

The results show that not only is the guar gum powder effective in reducing total serum cholesterol levels but is effective in reducing LDL cholesterol which, as discussed previously, forms the major part of the buildup on the arterial wall.

The formulation is also effective in lowering triglyceride levels which is also desirable to reduce the risk of heart disease. The average triglyceride reduction in the subjects when the guar gum drink mix was taken for six weeks is as follows :

## TABLE 2

| PARTICIPANT | TB | TA | % CHANGE |
|---|---|---|---|
| 1 | 137 | 92 | -32 |
| 2 | 400 | 189 | -52 |
| 3 | 261 | 238 | - 9 |
| 4 | 195 | 103 | -47 |
| 5 | 179 | - | - |
| 6 | 262 | 220 | -16 |
| 7 | 253 | 161 | -36 |
| 8 | 80 | 86 | +7.5 |
| 9 | 134 | 140 | +4.4 |
| 10 | 172 | 128 | -25 |
| 11 | 189 | 159 | -16 |
| 12 | 198 | 178 | -10 |
| 13 | 108 | 80 | -26 |

AVERAGE TRIGLYCERIDE REDUCTION    21.42%

TB = TRIGLYCERIDES BEFORE
TA = TRIGLYCERIDES AFTER 6 WEEKS

In order to retard the gelation of guar gum using citric acid, it was discovered that the more citric acid added to the powder percentage-wise, the greater the gelation time. For example, when 625 milligrams (0.625 g) of citric acid (12.5%) is blended with 4.375 grams of guar gum, and the resulting mixture is added to 100 milliliters of water, the mixture is still drinkable after five minutes. Addition of 4-8% by weight of ascorbic acid retards gelation even longer, making a total of 20.5% of the organic acid.

The following table illustrates that addition of a food-grade acid such as citric acid to powdered guar gum increases gelation time when the powder is mixed in water. For testing purposes, the mixture was agitated constantly. When the mixture is constantly agitated by a magnetic stirrer, the gelation time is speeded up. The gelation time is reduced by agitation because of increased hydration of the powder, and this results in faster gelation. Therefore, the following table indicates gelation times less than the five minutes stated in the preceding paragraph.

EP 0 316 146 A1

### TABLE 3

| WEIGHT OF CITRIC ACID g/100 ml $H_2O$ | PERCENT | WEIGHT OF GUAR GUM (grams) | GELATION TIME (seconds) |
|---|---|---|---|
| 0.00 | 00% | 5.0 | 49 |
| 0.25 | 5.0 | 4.75 | 65 |
| 0.625 | 12.5 | 4.375 | 79 |

It should be noted that, even with constant agitation, the times for gelation are extended long enough to allow for convenient ingestion after mixing.

The same results are obtained when using, instead of the citric acid, tartaric acid, phosphoric acid, ascorbic acid, or malic acid, the first introducing a definite grape flavor and the latter introducing a pleasant apple taste into the composition.

### Claims

1. A powder composition comprising guar gum and a food-grade organic acid.

2. A composition according to claim 1, wherein the amount of food-grade acid is sufficient to increase the gelation time of the composition when water is added thereto, e.g. 5 to 20.5% by weight of the guar gum.

3. A composition according to claim 1 or claim 2, wherein the food-grade acid comprises citric acid, ascorbic acid, tartaric acid, phosphoric acid or malic acid.

4. A composition according to claim 3, wherein the food-grade acid comprises citric acid.

5. A composition according to claim 4, comprising ascorbic acid in an amount of 4 to 8% by weight of the guar gum.

6. A composition according to any preceding claim, further comprising an orally-ingestible, non-toxic mineral salt capable of dissolution in gastric fluid.

7. A composition according to claim 6, wherein the salt is calcium carbonate, magnesium carbonate or potassium carbonate.

8. A composition according to any preceding claim, further comprising a flavouring agent, potassium citrate, a sweetener and a colourant.

9. An aqueous composition obtainable by mixing a powder composition according to any preceding claim with water.

10. A composition according to any preceding claim, for therapeutic use.

6

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 241 710 (U.SPECK) <br> * Claims; page 6, lines 29-33; page 7, lines 7-9 * | 1-10 | A 61 K 47/00 <br> A 61 K 31/715 |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 15-02-1989 | SCARPONI U. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)